# EUROPÄISCHE PATENTSCHRIFT

(11) **EP 2 461 749 B1**
(45) Veröffentlichungstag und Bekanntmachung des Hinweises auf die Patenterteilung: **03.12.2014**
(21) Anmeldenummer: 10742127.3
(22) Anmeldetag: 03.08.2010
(51) Int. Cl.: A61B 5/151

(54) **STEUEREINRICHTUNG FÜR EIN MEDIZINISCHES TESTSYSTEM**
Control device for a medical device
Dispositif de commande pour un appareil médical

(30) Priorität: 04.08.2009 EP 09167178
(43) Veröffentlichungstag der Anmeldung: 13.06.2012
(73) Patentinhaber: Roche Diagnostics GmbH, 68305 Mannheim (DE); F.Hoffmann-La Roche AG, 4070 Basel (CH)
(72) Erfinder: KUHR, Hans-Juergen, 68219 Mannheim (DE); HARTTIG, Herbert, 67434 Neustadt (DE); HAAR, Hans-Peter, 69168 Wiesloch (DE); LIST, Hans, 64754 Hesseneck-Kailbach (DE)
(74) Vertreter: Pfiz, Thomas
(86) Internationale Anmeldenummer: PCT/EP2010/061301
(87) Internationale Veröffentlichungsnummer: WO 2011/015585

(56) Entgegenhaltungen:
- WO-A1-2005/104949
- US-A1- 2008 021 490
- US-A1- 2009 192 409

## Beschreibung

Die Erfindung betrifft eine Steuereinrichtung für ein medizinisches Testsystem, das ein in Körpergewebe einstechbares Stechelement zum Sammeln von Körperflüssigkeit und einen Stechantrieb für das Stechelement aufweist, mit einem an dem Stechelement angeordneten Sensorelement für Körperflüssigkeit und einer mit dem Sensorelement gekoppelten Kontrolleinheit zur Erfassung eines Fehlerfalls beim Sammeln von Körperflüssigkeit. Die Erfindung betrifft weiter ein Testsystem mit einer solchen Steuereinrichtung.

Die Wiederholung eines Stechvorgangs zur besseren Blutgewinnung ist bei Stechhilfen mit Einweg-Lanzetten bekannt. Führt der erste Stich zu keinem ausreichenden Blutstropfen, so spannt der Anwender die Stechhilfe erneut und setzt mit derselben Lanzette einen zweiten, gegebenenfalls tieferen Einstich. Der Nachteil dieser mechanischen Stechvorrichtungen liegt in der Steuerung durch den Anwender, der letztendlich über eine Stichwiederholung entscheiden muss. Die verstrichene Zeit zwischen zwei Stichen ist entsprechend groß - typischerweise 10 bis 20 Sekunden - und kaum zu verkürzen.

Die Schrift US2008/0021490 A1 offenbart ein Stechgerät gemäß der Präambel von Anspruch 1. Mit elektrisch betriebenen Stechhilfen, bei denen das Spannen und/oder der eigentliche Stich durch elektro-mechanische Komponenten initiiert werden, ist eine schnellere Stichwiederholung denkbar. In der EP-A 1 982 653 wird vorgeschlagen, dass nach dem ersten Stich mit einem Stechelement eine Kontrolle des Blutgewinnungserfolges zwischengeschaltet wird. Dies soll es dem Anwender ein manuelles Aufnehmen einer Probe ermöglichen. Diese Art der Rückkopplung wird durch ein in das Blutgewinnungssystem integriertes Testelement erreicht, das mit dem aus dem Erststich gesammelten Blut einen Messwert erzeugen soll. Solche Blutzuckermesssysteme werden daher auch als "Integrierte Spot-Monitoring" (ISM)-Systeme bezeichnet. Solche ISM-Systeme müssen allerdings in der Lage sein, bei der Blutgewinnung die unterschiedlichsten Hauttypen sowie menschliche Fehlanwendungen zu berücksichtigen, ohne den Anwender zu überfordern oder zu beinträchtigen. Für Diabetiker ist im Rahmen einer Selbstkontrolle eine häufige Blutzuckerbestimmung unerlässlich. Dabei ist eine hohe Erfolgsrate wünschenswert, ohne dass Testmittel unnötig verbraucht werden.

Ausgehend hiervon liegt der Erfindung die Aufgabe zugrunde, die im Stand der Technik bekannten Einrichtungen weiter zu verbessern und eine erfolgreiche Testdurchführung mit hohem Komfort für den Anwender sicherzustellen.
Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch eine Art von Online-Detektion des Stecherfolgs bereits während des Stechvorgangs eine Wiederverwendung eines Stechelements mit integrierter Sammelstruktur für einen Zweitstich zu ermöglichen. Dementsprechend wird erfindungsgemäß vorgeschlagen, dass die Kontrolleinheit den Stechantrieb im Fehlerfall in weniger als 1 Sekunde zur automatischen Stichwiederholung ansteuert. Dieses Zeitfenster beruht auf der überraschenden Erkenntnis, dass ein Testerfolg durch Verdunstungsvorgänge beeinträchtigt werden kann. Aufgrund des kurzzeitigen automatischen Ablaufs kann somit eine Unterdosierung vermieden werden, ohne dass der Sammelvorgang behindert wird. Eine solche Stichwiederholung lässt sich dann mit einem einzigen disposiblen Stechelement mit integriertem Sensorelement durchführen, so dass teure Testmittel nicht ungewollt verschwendet werden. Zudem ist in dem Wiederholintervall keine zusätzliche Manipulation des Körpergewebes vorgesehen bzw. auch nicht erforderlich, so dass für den Anwender ein hoher Gebrauchskomfort resultiert. Der Erststich erfolgt in einer hin und her bzw. vor und zurück gehenden Stechbewegung, wobei das Stechelement vor dem Zweitstich ggf. vollständig bis zum Ausgangspunkt aus dem Körpergewebe herausgezogen wird, um einen einheitlichen Stechablauf zu ermöglichen.

Zur Berücksichtigung der aufgezeigten Randbedingungen beim Hauteinstich ist es vorteilhaft, wenn das Wiederholintervall weniger als 700 ms, bevorzugt weniger als 500 ms beträgt.

Für die möglichst schnelle Online-Detektion ist es von Vorteil, wenn das Sensorelement in fluidischer Verbindung mit einem vorzugsweise nicht in das Körpergewebe eingreifenden Abschnitt eines an dem Stechelement ausgebildeten Sammelkanals für Körperflüssigkeit angeordnet ist.

Vorteilhafterweise liefert das Sensorelement bei Benetzung mit Körperflüssigkeit ein Signal, das bevorzugt sowohl als Kontrollsignal für das Sammeln von Körperflüssigkeit als auch als Messsignal für einen Inhaltsstoff der Körperflüssigkeit auswertbar ist. Eine weitere Verbesserung auch hinsichtlich der Reaktionszeit ergibt sich dadurch, dass das Sensorelement bei einer Benetzung mit Körperflüssigkeit ein sich unstetig änderndes Steuersignal insbesondere in Form eines Remissionssprungs liefert.

Zur Verwirklichung kurzer Regelstrecken ist es günstig, wenn das Sensorelement über mindestens einen Signalleiter, vorzugsweise mindestens einen Lichtleiter während der Stechbewegung des Stechelements mit der Kontrolleinheit verbunden ist.

Vorteilhafterweise besitzt das Sensorelement zwei oder mehr im Abstand voneinander angeordnete Erfassungsbereiche zur Erfassung einer flächigen Beaufschlagung mit Körperflüssigkeit, so dass eine Unterdosierung am Messort gezielt erkennbar ist.

In diesem Zusammenhang ist es vorteilhaft, wenn die Kontrolleinheit eine ausbleibende oder eine nicht hinreichende Benetzung des Sensorelements mit Körperflüssigkeit vorzugsweise bereits während des Stechvorgangs als Fehlerfall erfasst, wobei ggf. unterschiedliche Abhilfemaßnahmen getroffen werden können.

Eine weitere Verbesserung der Benutzerfreundlichkeit lässt sich dadurch erreichen, dass die Kontrolleinheit über einen Hardware- oder Softwareschalter durch einen Benutzer des Testsystems deaktivierbar ist.

Dabei ist es auch von Vorteil, wenn die Kontrolleinheit einen Regelkreis aufweist, der Stechparameter wie Stechtiefe und Verweildauer des Stechelements im Körpergewebe für den Zweitstich automatisch anpasst.

Ein weiterer Aspekt der Erfindung liegt in einem Testsystem insbesondere für Blutzuckertests mit einem in Körpergewebe einstechbaren Stechelement zum Sammeln von Körperflüssigkeit und einem Stechantrieb für das Stechelement, wobei eine erfindungsgemäße Steuereinrichtung implementiert ist.

Für eine möglichst schnelle Stichwiederholung ist es vorteilhaft, wenn der Stechantrieb einen für einen Mehrfachstich dimensionierten Energiespeicher oder mehrere für jeweils einen Einzelstich dimensionierte Energiespeicher aufweist.

Denkbar ist es auch, dass der Stechantrieb einen kurzzeitig ohne Beschädigung überbestrombaren bürstenlosen Gleichstrommotor für eine direkte Übertragung der Stechbewegung aufweist.

Eine weitere Verbesserung des Integrationsgrades ergibt sich dadurch, dass das Sensorelement ein Nachweisfeld für einen Inhaltsstoff der Körperflüssigkeit aufweist, und dass der Inhaltsstoff sowie der Benetzungszustand des Nachweisfelds mit Körperflüssigkeit über eine einheitliche Messeinheit erfassbar sind.

Um die Probengewinnung zu optimieren, sollte das Stechelement ein Sammelvolumen von weniger als 100 nl zur Aufnahme von Körperflüssigkeit, insbesondere Blut aufweisen.

Günstig ist es auch, wenn das Stechelement einen vorzugsweise kapillaren Transportkanal für einen Transfer der Körperflüssigkeit auf das Sensorelement in einer Transferzeit von weniger als 0,3 s nach dem Hauteinstich aufweist.

Im Folgenden wird die Erfindung anhand der in der Zeichnung dargestellten Ausführungsbeispiele näher erläutert. Es zeigen:
- Fig. 1: ein medizinisches Testsystem mit einer Einrichtung zur Stichwiederholung für Blutzuckertests in einer schaubildlichen Darstellung;
- Fig. 2: eine weitere Ausführungsform eines Testgeräts;
- Fig. 3: ein Diagramm der zeitlichen Messabweichung in Verbindung mit verschiedenen Schwellen für die Stichwiederholung;
- Fig. 4: den Zeitverlauf eines Messsignals bei der photometrischen Blutzuckermessung;
- Fig. 5: den Ablauf der Stichwiederholung in einem Flussdiagramm.

Die in der Zeichnung dargestellten Testsysteme 10 ermöglichen als Handgerät die automatisierte Probengewinnung und Messwerterfassung zur Blutzuckerbestimmung. Zu diesem Zweck ist eine als Einwegartikel eingesetzte Testeinheit 12 mittels eines Stechantriebs 14 in einer hin und hergehenden Stechbewegung zur Ausführung eines Hauteinstichs antreibbar, wobei die Blutprobengewinnung mittels einer Kontrolleinheit 16 überwacht wird und vor Ort eine Messwertauswertung und -anzeige für den Benutzer erfolgt. Durch einen nach dem hin- und hergehenden Erststich kurzzeitig erfolgenden Zweitstich kann die Erfolgsquote nach einem fehlerhaften Sammelvorgang deutlich verbessert werden.

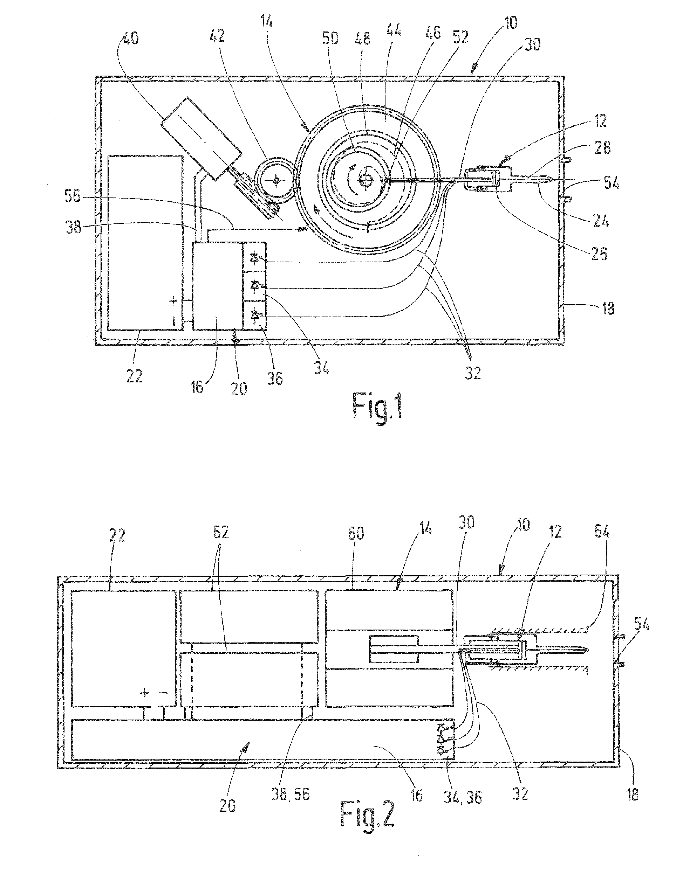

Fig. 1 veranschaulicht nach Art eines Blockschaltbilds das Handgerät mit Gehäuse 18, Stechantrieb 14, Messeinrichtung 20 und Energieversorgung 22. Daneben bilden die vorzugsweise in einem Magazin einwechselbaren disposiblen Testeinheiten 12 einen Bestandteil des analytischen Testsystems 10.

Jede Testeinheit 12 umfasst ein Stechelement 24 und ein daran angeordnetes Sensorelement 26, das nach einer Analyse verbraucht ist. Das als Flachformteil ausgebildete Stechelement 24 weist im Bereich seiner Spitze einen rillenförmigen, einseitig offenen Kapillarkanal 28 als Sammelstruktur für Körperflüssigkeit auf. Der Kapillarkanal 28 besitzt ein Aufnahmevolumen von unter 100 nl und mündet proximal auf einem trockenchemischen Nachweisfeld des Sensorelements 26, das rückseitig an die reflexionsphotometrisch arbeitende Messeinrichtung 20 ankoppelbar ist. Die U-förmige Basis des Stechelements 12 lässt sich an einen hin und her bewegbaren Stößel 30 des Stechantriebs 14 andocken, wobei zugleich über Lichtleiter 32 eine optische Verbindung zu der Messeinrichtung 20 geschaffen wird.

Für die reflektometrische Erfassung von Messsignalen weist die Messeinrichtung eine LED 34 als Lichtquelle und zwei Photodioden 36 als Lichtempfänger auf. Die Photodioden 36 sind über die Lichtleiter 32 auf unterschiedliche Erfassungsbereiche bzw. Spots des Nachweisfelds des Sensorelements 26 ausgerichtet. Die empfangenen Messsignale lassen sich in der mikroprozessorgestützten Messeinrichtung 20 sowohl qualitativ hinsichtlich des Vorhandenseins einer Blutprobe am Nachweisfeld als auch quantitativ hinsichtlich des Glukosegehalts auswerten, wie es weiter unten näher erläutert wird. Ausgangsseitig ist eine Stelleinheit 38 vorgesehen, um den Stechantrieb 14 anzusteuern.

Der Stechantrieb 14 umfasst einen Gleichstrommotor 40, ein Getriebe 42, einen Spannrotor 44, eine Spiralfeder 46, einen Kulissenrotor 48 mit exzentrischer Steuerkulisse in Form einer Kreisnut 50, in die ein rückwärtiger Steuerzapfen 52 des Stößels 30 eingreift.

Der Spannrotor 44 spannt bei drehfest gehaltenem Kulissenrotor 48 die Spiralfeder 46 als Energiespeicher. Zur Auslösung eines Hauteinstichs wird der Kulissenrotor 48 für eine Umdrehung freigegeben, so dass der Stößel 30 eine hin- und hergehende Stechbewegung auf das Stechelement 24 überträgt. Dieses dringt dabei mit seiner Spitze in die Haut eines an die Gehäuseöffnung 54 angepressten Körperteils, z.B. einer Fingerkuppe ein und nimmt in dem distalen Abschnitt des Sammelkanals 28 das in der Stichwunde freigesetzte Kapillarblut bzw. ggf. auch Gewebeflüssigkeit auf. Ggf. kann durch Betätigung von geeigneten Stellelementen 56 beispielsweise in Form von Steueranschlägen und Steuernocken, wie sie aus der EP 1669028 an sich bekannt sind, kurzzeitig ein Zweitstich ausgelöst werden, wobei der Kulissenrotor 48 mit der Restspannung der Spiralfeder 46 eine erneute Umdrehung für eine zweite hin- und hergehende Stechbewegung ausführt. Zwischen Erst- und Zweitstich wird das Stechelement 24 ggf. vollständig aus dem Körpergewebe heraus zurückgezogen.

In der Ausführungsform nach Fig. 2 sind gleiche oder ähnliche Teile mit den zuvor verwendeten Bezugszeichen versehen. Dieses Beispiel unterscheidet sich im Wesentlichen nur dadurch, dass anstelle eines mechanischen Antriebsstrangs für die Stechbewegung ein Linearmotor 60 als Direktantrieb für die über den Stößel 30 angekoppelte Stecheinheit 12 zum Einsatz kommt, und dass zwei Energiespeicher 62 in Form von Kondensatoren die notwendige Antriebsenergie für jeweils eine hin und hergehende Stechbewegung parallel bereitstellen, ohne dass die Batterie 22 sonderlich lieferfähig sein muss. Zur Begrenzung auf eine lineare Stechbewegung ist eine Führung 64 vorgesehen, die in Fig. 1 der besseren Übersichtlichkeit halber nicht gezeigt ist.

Es ist auch vorstellbar, dass das System abwechselnd zwischen den beiden Energiespeichern 62 hin und herschaltet. Der Vorteil bestünde darin, dass im Normalfall (kein Zweitstich erforderlich) immer einer der beiden Speicher bereits aufgefüllt ist. Somit kann das System beim Ein- oder Ausschalten mit weniger Aufwand den restlichen Speicher füllen. Elektrische oder elektromagnetische Energiespeicher sind hierbei besonders vorteilhaft, da sie über einen einfachen elektrischen Schaltkreis angesprochen und zugeschaltet werden können.

Die Beispiele nach Fig. 1 und 2 erlauben eine Stichwiederholung in einem Zeitintervall von weniger als 1 Sekunde, wenn die Kontrolleinrichtung 16 eine unzureichende Probenaufnahme detektiert. In einem nicht eigens gezeigten weiteren Konzept kommt ein bürstenloser Gleichstrommotor zum Einsatz, wie er beispielsweise für den Antrieb von CD- oder Festplattenlaufwerken verwendet wird. Ein solcher Motor hat eine kleine, flache Bauform und eignet sich daher gut für eine Unterbringung auf einer Platine. Seine weiteren Vorzüge liegen im hohen Wirkungsgrad und seiner Toleranz gegenüber kurzzeitigem Überbestromen. Hierbei kann etwa das Zehnfache der Dauermotorleistung aufgebracht werden, so dass eine Stichwiederholung in dem genannten Zeitintervall von weniger als 1 Sekunde möglich ist.

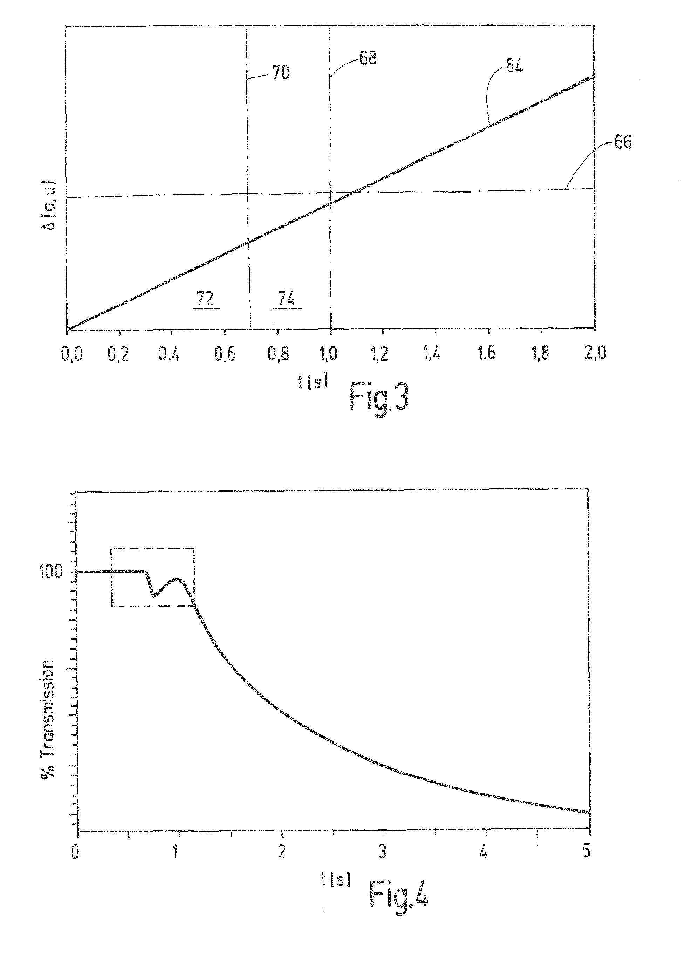

Das Diagramm nach Fig. 3 verdeutlicht verschiedene überraschende Zusammenhänge bei der Stichwiederholung. Durch Wasserverdunstung aus der Probe beim Transport von der Wunde zum Testfeld erhöht sich die Messungenauigkeit Δ. Je länger Transportweg 28 ist und je langsamer der Probentransfer erfolgt, desto mehr verdunstet Wasser aus der Probenflüssigkeit und verfälscht somit das Messergebnis etwa linear über der Zeit (ansteigende Kurve 64). Die Grenzwertschwelle 66 gibt hierbei eine noch akzeptable Messwertverfälschung an ("Imprecision Threshold").

Ist der Probentransfer nach dem Erststich vorzeitig zum Stillstand gekommen, so ist einen Nachdosierung nur eine begrenzte Zeit möglich, da ansonsten die Blutfront in der Kapillare 28 eingetrocknet ist und keinen Weitertransport mehr zulässt. Erkenntnisse der Anmelder weisen darauf hin, dass eine Unterbrechung des Probentransfers um 2 s zu einem irreversiblen Stillstand der Blutfront führt, während bei einer kürzeren Unterbrechung von weniger als 1 s die Blutfront wieder losläuft. Diese zeitliche Obergrenze ("Afterdosing Threshold") ist in Fig. 3 mit 68 bezeichnet.

Die Rückkopplung bei der Fehlerdetektion sollte so schnell sein, dass der Anwender von einer erforderlichen Stichwiederholung nichts bemerkt. Diese Erkennungsschwelle 70 ("Recognition Threshold") liegt bei etwa 0,7 s.

Im Idealfall wird also das Messfenster 72 genutzt, in dem alle Grenzen 66, 68, 70 eingehalten werden. Das größere Zeitfenster 74 bezieht den nutzbaren Bereich ein, der die Erkennbarkeit der Stichwiederholung durch den Anwender unberücksichtigt lässt.

Hierfür sollte durch geeignete Ausgestaltung des Kanals 28 (insbesondere Länge, Kapillarität und Benetzbarkeit) gewährleistet sein, dass die Dauer für den Probentransport weniger als 0,3 s beträgt. Die optische Auswertung des Nachweisfelds des Sensorelements 26 mittels Remissionsmessung erlaubt zudem eine zügige Kontrolle des Benetzungszustandes, da das benetzte Feld einen sofortigen Remissionssprung herbeiführt und erst danach eine Farbveränderung durchläuft, die sich zur Bestimmung des Blutzuckerwertes heranziehen lässt.

Das gestrichelte Fenster in Fig. 4 zeigt den Bereich einer Messkurve auf, der für diese Fallentscheidung herangezogen werden kann. Der auf 100% normierte Startwert entspricht dem Messwert zum Zeitpunkt des Erststiches bei t = 0s. In diesem Beispiel fällt nach 0,7 s der Messwert infolge der Nachweisfeldbenetzung signifikant ab, was hier als Remissionssprung bezeichnet wird. Nach einer kurzzeitigen Erholung beginnt die Farbenentwicklung der Testchemie und die Kurve fällt stetig. Somit ist es also möglich, kurzzeitig nach dem Erststich zu entscheiden, ob eine ausreichende Menge Blut auf dem Sensorelement angelangt ist oder ob ein Wiederholungsstich durchgeführt werden soll.

Die Fallentscheidung zum Zweitstich wird zweckmäßigerweise einem system-internen Regelkreis, beispielsweise einer Software-Routine überlassen, da dieser schneller und fehlerfreier entscheiden kann als der Anwender selbst. Wahlweise kann ein solcher Automatismus über einen nicht gezeigten Hardware- bzw. Softwareschalter deaktiviert werden.

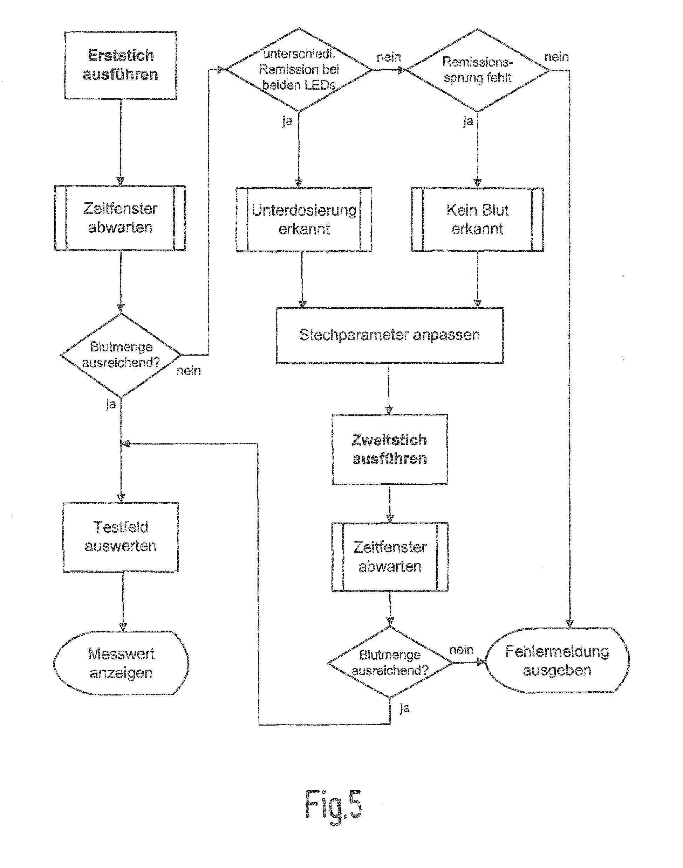

Wie aus dem Flussdiagramm Fig. 5 exemplarisch hervorgeht, lässt sich der Stechablauf mittels der Kontrolleinheit 16 je nach Fehlerfall steuern. Falls beim ersten Stich überhaupt kein Blut gewonnen wurde, so wird dies über den fehlenden Remissionssprung im vorgegebenen Zeitfenster erkannt. In diesem Fall ist es wahrscheinlich, dass die Einstechtiefe in die Haut nicht ausreichend war, und das System kann einen Zweitstich mit erhöhter Eindringtiefe vorbereiten. Es ist jedoch auch denkbar, das dynamische Stechprofil entsprechend zu verändern, beispielsweise durch eine längere Verweildauer des Stechelements 24 im Gewebe.

Ist die beim Erststich gewonnene Blutmenge zu gering, so führt dies auf dem Nachweisfeld im vorgegebenen Zeitfenster zwar zu einem Remissionssprung, jedoch ist die benetzte Fläche sehr klein. Dieser Fall kann mit zwei seitlich versetzten Erfassungsbereichen erkannt werden, in denen sich bei Unterdosierung eine unterschiedliche Remission, bei ausreichender Dosierung hingegen eine übereinstimmende Remission ergibt. Ist eine Unterdosierung erkannt worden, kann das System einen Zweitstich vorbereiten. Die Stechparameter können entsprechend korrigiert werden, müssen aber nicht den gleichen Regeln unterliegen wie im oben beschriebenen Fall. Gegebenenfalls ist gerade bei Blut-Unterdosierung ein Wiederholungsstich mit den aus dem Erststich verwendeten Parametern bereits ausreichend. Die Abfrage nach der Blutmenge sollte auch bei einem Zweitstich in weniger als 1 s abgeschlossen sein, damit der Anwender seinen Finger von der Stechöffnung 54 entfernen kann. Die weitere Auswertung und anschließende Messwertanzeige kann dann in einem größeren Zeitfenster von mehreren Sekunden erfolgen.

## Patentansprüche

1. Steuereinrichtung für ein medizinisches Testsystem (10), das ein in Körpergewebe einstechbares Stechelement (24) zum Sammeln von Körperflüssigkeit und einen Stechantrieb (14) für das Stechelement (24) aufweiset, mit einem an dem Stechelement (24) angeordneten Sensorelement (26) für Körperflüssigkeit und einer mit dem Sensorelement (26) gekoppelten Kontrolleinheit (16) zur Erfassung eines Fehlerfalls beim Sammeln von Körperflüssigkeit, dadurch gakennzeichnet, dass die Kontrolleinheit (16) den Stechantrieb (14) im Fehlerfall nach einer hin und hergehenden ersten Stechbewegung in einem Wiederholintervall von weniger als 1 Sekunde zur automatischen Stichwiederholung für eine zweite hin und hergehende Stechbewegung ansteuert.

2. Steuereinrichtung nach Anspruch 1, **dadurch gekennzeichnet, dass** das Wiederholintervall weniger als 700 ms, bevorzugt weniger als 500 ms beträgt,

3. Steuereinrichtung nach Anspruch 1 oder 2, **dadurch gekennzeichnet, dass** das Sensorelement (26) in fluidischer Verbindung mit einem vorzugsweise nicht in das Körpergewebe eingreifenden Abschnitt eines an dem Stechelement (24) ausgebildeten Sammelkanals (28) für Körperflüssigkeit angeordnet ist.

4. Steuereinrichtung nach einem der Ansprüche 1 bis 3, **dadurch gekennzeichnet, dass** das Sensorelement (26) bei einer Benetzung mit Körperflüssigkeit ein sich unstetig änderndes Steuersignal insbesondere in Form eines Remissionssprungs liefert.

5. Steuereinrichtung nach einem der Ansprüche 1 bis 4, **dadurch gekennzeichnet, dass** das Sensorelement (26) über mindestens einen Signalleiter (32), vorzugsweise mindestens einen Lichtleiter während der Stechbewegung des Stechelements (24) mit der Kontrolleinheit (16) verbunden ist.

6. Steuereinrichtung nach einem der Ansprüche 1 bis 5, **dadurch gekennzeichnet, dass** das Sensorelement (26) zwei oder mehr im Abstand voneinander angeordnete Erfassungsbereiche zur Erfassung einer flächigen Beaufschlagung mit Körperflüssigkeit aufweist.

7. Steuereinrichtung nach einem der Ansprüche 1 bis 6, **dadurch gekennzeichnet, dass** die Kontrolleinheit (16) eine ausbleibende oder nicht hinreichende Benetzung des Sensorelements (26) mit Körperflüssigkeit als Fehlerfall erfasst.

8. Steuereinrichtung nach einem der Ansprüche 1 bis 7, **dadurch gekennzeichnet, dass** die Kontrolleinheit (16) über einen Hardware- oder Softwareschalter durch einen Benutzer des Testsystems deaktivierbar ist.

9. Steuereinrichtung nach einem der Ansprüche 1 bis 8, **dadurch gekennzeichnet, dass** die Kontrolleinheit (16) einen Regelkreis aufweist, der Stechparameter wie Stechtiefe und Verweildauer des Stechelements (24) im Körpergewebe für den Zweitstich automatisch anpasst.

10. Testsystem insbesondere für Blutzuckertests mit einem in Körpergewebe einstechbaren Stechelement (24) zum Sammeln von Körperflüssigkeit und einem Stechantrieb (14) für das Stechelement (24), **gekennzeichnet durch** eine Steuereinrichtung nach einem der vorhergehenden Ansprüche.

11. Testsystem nach Anspruch 10, **dadurch gekennzeichnet, dass** der Stechantrieb (14) einen für einen Mehrfachstich dimensionierten Energiespeicher (46) oder mehrere für jeweils einen Einzelstich dimensionierte Energiespeicher (62) aufweist.

12. Testsystem nach Anspruch 10, **dadurch gekennzeichnet, dass** der Stechantrieb (14) einen kurzzeitig überbestrombaren bürstenlosen Gleichstrommotor aufweist.

13. Testsystem nach einem der Ansprüche 10 bis 12, **dadurch gekennzeichnet, dass** das Sensorelement (26) ein Nachweisfeld für einen Inhaltsstoff der Körperflüssigkeit aufweist, und dass der Inhaltsstoff sowie der Benetzungszustand des Nachweisfelds mit Körperflüssigkeit über eine einheitliche Messeinheit (20) erfassbar sind.

14. Testsystem nach einem der Ansprüche 10 bis 13, **dadurch gekennzeichnet, dass** das Stechelement (24) ein Sammelvolumen von weniger als 100 nl zur Aufnahme von Körperflüssigkeit, insbesondere Blut aufweist.

15. Testsystem nach einem der Ansprüche 10 bis 14, **dadurch gekennzeichnet, dass** das Stechelement (24) einen vorzugsweise kapillaren Sammelkanal (28) für einen Transfer der Körperflüssigkeit auf das Sensorelement (26) in einer Transferzeit von weniger als 0,3 s nach dem Hauteinstich aufweist.

## Claims

1. Control device for a medical test system (10) which has a lancing element (24) that can be inserted into body tissue to collect body fluid and a lancing drive (14) for the lancing element (24), comprising a sensor element (26) for body fluid arranged on the lancing element (24) and a control unit (16) coupled to the sensor element (26) for detecting a fault when collecting body fluid, **characterized in that** in case of a fault after an reciprocating first lancing movement the control unit (16) actuates the lancing drive (14) in a repetition interval of less than 1 second for an automatic puncture repetition in a second reciprocating lancing movement.

2. Control device according to claim 1, **characterized in that** the repetition interval is less than 700 ms, preferably less than 500 ms.

3. Control device according to claim 1 or 2, **characterized in that** the sensor element (26) is arranged in fluidic connection with a section of a collecting channel (28) for body fluid formed on the lancing element (24) which preferably does not engage into the body tissue.

4. Control device according to one of the claims 1 to 3, **characterized in that** the sensor element (26) generates a control signal that changes discontinuously in particular in the form of a step-change in reflectance when it is wetted with body fluid.

5. Control device according to one of the claims 1 to 4, **characterized in that** the sensor element (26) is connected to the control unit (16) by means of at least one signal conductor (32) preferably at least one light guide during the lancing movement of the lancing element (24).

6. Control device according to one of the claims 1 to 5, **characterized in that** the sensor element (26) has two or more detection areas arranged spaced apart from one another to detect a two-dimensional loadwith body fluid.

7. Control device according to one of the claims 1 to 6, **characterized in that** the control unit (16) detects an absence of wetting or an inadequate wetting of the sensor element (26) with body fluid as a fault.

8. Control device according to one of the claims 1 to 7, **characterized in that** the control unit (16) can be deactivated by a user of the test system by means of a hardware or software switch.

9. Control device according to one of the claims 1 to 8, **characterized in that** the control unit (16) has a control loop which automatically adapts the lancing parameters for the second puncture such as lancing depth and dwell time of the lancing element (24) in the body tissue.

10. Test system, in particular for blood sugar tests comprising a lancing element (24) that can be inserted into body tissue to collect body fluid and a lancing drive (14) for the lancing element (24), **characterized by** a control device according to one of the previous claims.

11. Test system according to claim 10, **characterized in that** the lancing drive (14) has an energy store (46) dimensioned for a multiple puncture or several energy stores (62) each dimensioned for a single puncture.

12. Test system according to claim 10, **characterized in that** the lancing drive (14) has a brushless direct current motor that can be temporary subjected to an overcurrent.

13. Test system according to one of the claims 10 to 12, **characterized in that** the sensor element (26) has a detection field for a component of the body fluid, and that the component as well as the wetting state of the detection field with body fluid can be detected by means of a unitary measuring unit (20).

14. Test system according to one of the claims 10 to 13, **characterized in that** the lancing element (24) has a collecting volume of less than 100 nl to collect body fluid, in particular blood.

15. Test system according to one of the claims 10 to 14, **characterized in that** the lancing element (24) has a preferably capillary collecting channel (28) to transfer the body fluid onto the sensor element (28) in a transfer time of less than 0.3 s after the skin puncture.

## Revendications

1. Dispositif de commande pour un système de test médical (10) qui comprend un élément piquant (24) permettant d'inciser un tissu corporel pour collecter un fluide corporel et un mécanisme d'entraînement de piqûre (14) pour l'élément piquant (24), avec un élément détecteur (26) disposé sur l'élément piquant (24) pour détecter le fluide corporel et une unité de contrôle (16) couplée à l'élément détecteur (26) pour détecter une erreur lors de la collecte du fluide corporel, **caractérisé en ce que** l'unité de contrôle (16) commande en cas d'erreur, après un premier mouvement de va-et-vient, une répétition automatique de piqûre du mécanisme d'entraînement de piqûre (14) pendant un intervalle de répétition inférieur à une seconde pour un second mouvement de va-et-vient.

2. Dispositif de commande selon la revendication 1, **caractérisé en ce que** l'intervalle de répétition est inférieur à 700 ms, de préférence inférieur à 500 ms.

3. Dispositif de commande selon la revendication 1 ou 2, **caractérisé en ce que** l'élément détecteur (26) est disposé de façon à être en communication fluidique avec une partie, de préférence non engagée dans le tissu corporel, d'un canal collecteur (28) de fluide corporel formé sur l'élément piquant (24).

4. Dispositif de commande selon l'une des revendications 1 à 3, **caractérisé en ce que** l'élément détecteur (26), lorsqu'il est humecté par le fluide corporel, fournit un signal de commande variant de façon discontinue, en particulier sous la forme d'un saut de réflectance.

5. Dispositif de commande selon l'une des revendications 1 à 4, **caractérisé en ce que** pendant le mouvement de piqûre de l'élément piquant (24), l'élément détecteur (26) est relié à l'unité de contrôle (16) par au moins un conducteur de signal (32), de préférence au moins un conducteur de lumière.

6. Dispositif de commande selon l'une des revendications 1 à 5, **caractérisé en ce que** l'élément détecteur (26) présente deux zones de détection ou plus disposées à distance l'une de l'autre pour la détection d'une mise en contact sur toute la surface avec le fluide corporel.

7. Dispositif de commande selon l'une des revendications 1 à 6, **caractérisé en ce que** l'unité de contrôle (16) détecte comme erreur un non-humectage ou un humectage insuffisant de l'élément détecteur (26) par le fluide corporel.

8. Dispositif de commande selon l'une des revendications 1 à 7, **caractérisé en ce que** l'unité de contrôle (16) peut être désactivée au moyen d'un interrupteur matériel ou logiciel par un utilisateur du système de test.

9. Dispositif de commande selon l'une des revendications 1 à 8, **caractérisé en ce que** l'unité de contrôle (16) comporte un circuit de régulation qui adapte automatiquement les paramètres de piqûre pour la seconde piqûre, telle que la profondeur de piqûre ou la durée de séjour de l'élément piquant (24) dans le tissu corporel.

10. Système de test notamment pour tests de glycémie, comprenant un élément piquant (24) permettant d'inciser un tissu corporel pour collecter un fluide corporel et un mécanisme d'entraînement de piqûre (14) pour l'élément piquant (24), **caractérisé par** un dispositif de commande selon l'une des revendications précédentes.

11. Système de test selon la revendication 10, **caractérisé en ce que** le mécanisme d'entraînement de piqûre (14) comprend un accumulateur d'énergie (46) dimensionné pour une piqûre multiple ou plusieurs accumulateurs d'énergie (62) prévus chacun pour une piqûre simple.

12. Système de test selon la revendication 10, **caractérisé en ce que** le mécanisme d'entraînement de piqûre (14) comprend un moteur à courant continu, sans balais, pouvant être alimenté en courant de surcharge sur une courte durée.

13. Système de test selon l'une des revendications 10 à 12, **caractérisé en ce que** l'élément détecteur (26) comporte une zone de détection pour un composant du fluide corporel et **en ce que** le composant ainsi que l'état d'humectage de la zone de détection par le fluide corporel peuvent être détectés au moyen d'une unité de mesure (20) uniforme.

14. Système de test selon l'une des revendications 10 à 13, **caractérisé en ce que** l'élément piquant (24) possède un volume de collecte inférieur à 100 nl pour recevoir le fluide corporel, en particulier du sang.

15. Système de test selon l'une des revendications 10 à 14, **caractérisé en ce que** l'élément piquant (24) comporte un canal collecteur (28), de préférence capillaire, pour le transfert du fluide corporel sur l'élément détecteur (26) en un temps de transfert inférieur à 0,3 s après l'incision de la peau.
